# EUROPEAN PATENT SPECIFICATION

(11) **EP 1 492 764 B1**
(45) Date of publication and mention of the grant of the patent: **28.06.2006**
(21) Application number: 03745340.4
(22) Date of filing: 28.03.2003
(51) Int. Cl.: C07C 303/22, C07C 309/66

(54) **PROCESS**
VERFAHREN
PROCEDE

(30) Priority: 02.04.2002 SE 0201005
(43) Date of publication of application: 05.01.2005
(73) Proprietor: AstraZeneca AB, 151 85 Södertälje (SE)
(72) Inventor: LARSSON, Maria, S-151 85 Sodertalje (SE)
(86) International application number: PCT/GB2003/001395
(87) International publication number: WO 2003/082812

(56) References cited:
- WO-A-99/62872
- R.W. KIERSTEAD, ET AL.: "beta1-Selective adrenoceptor antagonists. 1. Synthesis and beta-adrenergic blocking activity of a series of binary (aryloxy)propanolamines" JOURNAL OF MEDICINAL CHEMISTRY, vol. 26, no. 11, 1983, pages 1561-1569, XP002922416 AMERICAN CHEMICAL SOCIETY, WASHINGTON, DC, US ISSN: 0022-2623
- J.F.W. KEANA, ET AL.: "New mono- and difunctional 2,2,5,5-tetramethylpyrrolidine- and delta3-pyrroline-1-oxyl nitroxide spin labels" CANADIAN JOURNAL OF CHEMISTRY, vol. 60, no. 12, 15 June 1982 (1982-06-15), pages 1439-1447, XP002256848 NATIONAL RESEARCH COUNCIL, OTTAWA, CA

## Description

The present invention relates to an improved process for the preparation of the compound 2-ethoxy-3-[4-(2-{4-methanesulfonyloxyphenyl}ethoxy)phenyl]propanoic acid, as shown in formula Ia below 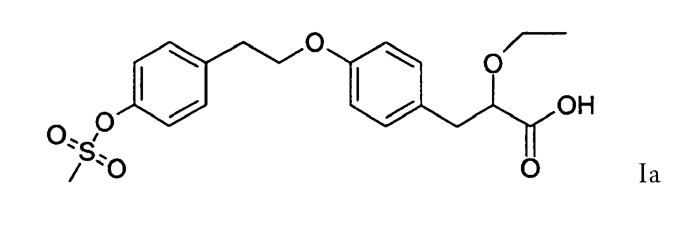 or the (R) or the (S) enantiomer thereof, or a pharmaceutically-acceptable salt thereof, and solvates thereof.

The above compound is intended for therapeutic use in the Insulin Resistance Syndrome (IRS) including type 2 diabetes mellitus, which refers to a cluster of manifestations including insulin resistance with accompanying hyperinsulinaemia, possible type 2 diabetes mellitus, arterial hypertension, central (visceral) obesity, dyslipidaemia observed as deranged lipoprotein levels typically characterised by elevated VLDL (very low density lipoproteins), small dense LDL particles and reduced HDL (high density lipoprotein) concentrations and reduced fibrinolysis.

Recent epidemiological research has documented that individuals with insulin resistance run a greatly increased risk of cardiovascular morbidity and mortality, notably suffering from myocardial infarction and stroke. In type 2 diabetes mellitus atherosclerosis related conditions cause up to 80% of all deaths.

In clinical medicine there is awareness of the need to increase the insulin sensitivity in IRS suffering patients and thus to correct the dyslipidaemia which is considered to cause the accelerated progress of atherosclerosis. However, currently this is not a universally well defined disease.

The compound of formula I is disclosed in PCT Publication Number WO99/62872. Two alternative processes are disclosed for the preparation of the compound of formula I in Examples 1 and 2 of the application. We have discovered an improvement in relation to one of the processes disclosed.

Specifically we have found an improved method for the synthesis of the penultimate esterified intermediate, the final step of the reaction being the conversion of the ester group into the acid of the final product.

Accordingly the present invention provides a process for the preparation of a compound of formula I 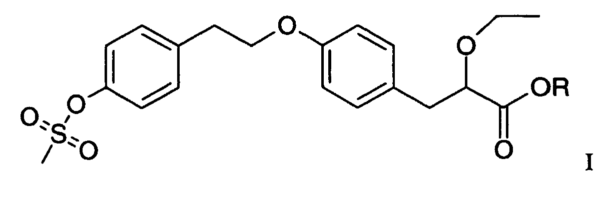 in which R represents H or an acid protecting group which comprises reacting a compound of formula II 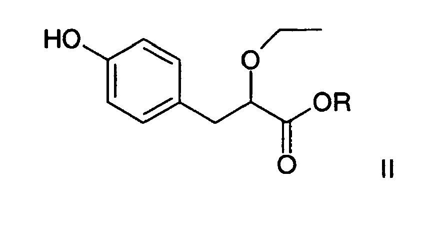 in which R is as previously defined with a compound of formula III 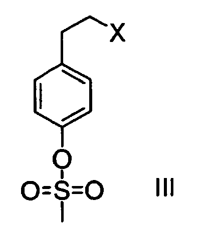 wherein X is a suitable leaving group in the presence of a base using water as the diluent.

Suitably the process is carried out at a temperature in the range 0°C to 250°C and preferably in the range 50°C to 150°C. More preferably the process is carried out at a temperature in the range of 80°C to 130°C and most preferably in the range of 90°C to 110°C.

The term " acid protecting group" means that the acid is protected from reaction by forming a suitable acid derivative such as an ester or amide or by other means of protection of carboxylic acid groups known in the art. Examples of suitable means of protection and acid derivatives (as well as means of formation and eventual deprotection), may be found in T.W. Greene and P.G.M. Wuts, "Protective Groups in Organic Synthesis", Third Edition, John Wiley & Sons, New York, 1999. The nature of the ester is not important in the performance of the process since its function is to act as a protecting group. The improvements relate to the application of phase transfer catalysis to the process.
Preferably R is H, benzyl or a (1-4C)alkyl group, such as methyl, ethyl or propyl. More preferably R is a (1-4C)alkyl group. Most preferably R is ethyl.

Suitably X is halo, for example bromo, chloro or iodo, an optionally substituted phenylsulfonyloxy group, in particular (4-methylphenyl)sulfonyloxy group or 2,4,6-triisopropylphenylsulfonyloxy group or an alkylsulphonyloxy group for example methanesulphonyloxy. Preferably X is a methanesulphonyloxy group.

Suitable bases include carbonates, hydrogen carbonates or hydroxides particularly of alkali metals. Preferably the base is sodium carbonate, sodium hydrogen carbonate, potassium carbonate or potassium hydrogen carbonate.

Suitably the molar ratio of the compound of formula III to the compound of formula II is in the range of 0.5 to 10 , preferably in the range 0.8 to 4 and more preferably in the range of 1.0 to 3 and most preferably is in the range of 1.2 to 1.8 for example 1.2 to 1.6.

Suitably the molar ratio of the base to the compound of formula II is in the range of 0.5 to 10 , preferably in the range 0.8 to 7 for example 0.8 to 4 and more preferably in the range of 1.0 to 5 and most preferably is in the range of 1.2 to 4.2.

The process of the invention has the following advantages. The reaction times are more rapid than the reactions known in the prior art and therefore the process is less costly to run. In addition the process gives higher yields and the product is of a higher purity than previously disclosed processes for the preparation of the compound of formula I. Also, it is a considerable advantage in terms of waste disposal, environmental reasons and cost to use water as the diluent instead of an organic liquid. Further, the process is consistently reproducible and robust.

Converting the acid ester derivative may be accomplished simply by hydrolysis (acidic or alkaline or enzymatic) of the ester to the acid, such a step being known to the skilled person, such as described in the examples below and in Example 2 i) of WO99/62872.

In another aspect the present invention provides a process for the preparation of a compound of formula I 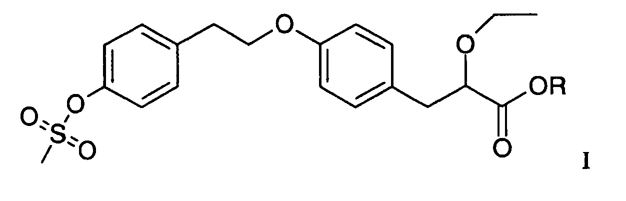 in which R represents H which comprises reacting a compound of formula II 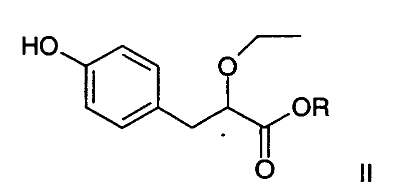 in which R represents an acid protecting group with a compound of formula III 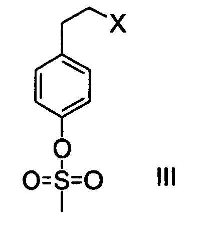 wherein X is a suitable leaving group in the presence of a base and using water as the diluent to give a compound of formula I in which R is an acid protecting group and then removing the protecting group to give a compound of formula I in which R is H.

In a preferred aspect the protecting group is an ester and the protecting group removal step comprises a hydrolysis step. The hydrolysis step may be acid or base catalysed. Preferably the base is lithium hydroxide. Optionally an organic liquid may be present in the hydrolysis step for example acetone, 2-butanone, methanol, ethanol, tetrahydrofuran or dioxane.

In a preferred aspect the process provides the S-enantiomer of the compound of formula I in which R is H by using the S-enantiomer of the compound of formula II in which R represents H or an acid protecting group followed by hydrolysis when R is an acid protecting group.

The compound of formula I in which R is H may be purified by recrystallisation. Suitable recrystallisation solvents include one or more of the following ethanol, water, isopropyl acetate, isopropanol, isooctane and toluene.

The invention is illustrated by the following non-limiting examples.

### Abbreviations

EtOAc = ethyl acetate
HPLC = high-pressure liquid chromatography
i-PrOAc = isopropyl acetate
EtOH= ethanol

### Preparation of Startine Material

2-(4-Methanesulfonyloxyphenyl)ethylmethanesulfonate was prepared as described in WO99/62872.

### Example 1 (S)-2-ethoxy-3-[4-(2-{4-methanesulfonyloxyphenyl}ethoxy)phenyl] propanoic acid

A mixture of 2-(4-methanesulfonyloxyphenyl)ethylmethanesulfonate (9.88 g / 33.57mmol), ethyl (S)-2-ethoxy-3-(4-hydroxyphenyl)propanoate (5.0 g / 20.98 mmol), Na₂CO₃ (2.96 g / 27.9 mmol eq) and water (10ml) was boiled under reflux with vigorous stirring for 4.5 h. The mixture was cooled to 2°C and acetone (13.5ml) was added. The mixture containing ethyl (S)-2-ethoxy-3-[4-(2-{4-methanesulfonyloxyphenyl}ethoxy)phenyl] propanoate was allowed to warm up to ambient temperature. The acetone solution was kept at Tᵢ = 25 °C in the reactor. LiOHxH₂O powder (1.144 g / 27.27mmol) dissolved in water (20ml) was charged continuously over 30 minutes under vigorous stirring at 25±5 °C. The reaction was continued for 7 hours. The reaction was quenched by addition of EtOAc (1.5 ml) at Tᵢ = 25±5 °C. Acetic acid was added to the water mixture after phase-separation and the pH was corrected to 1-3 using sulphuric acid in water. The crude title compound was crystallized by seeding. The identity of the product was confirmed by HPLC and gave a relative area of 76%.

## Claims

1. A process for the preparation of a compound of formula I 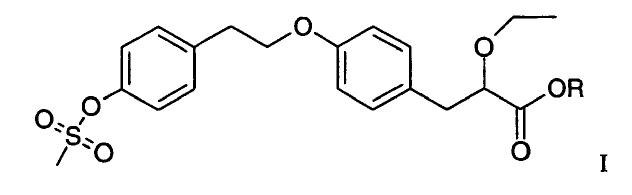 in which R represents H or an acid protecting group which comprises reacting a compound of formula II 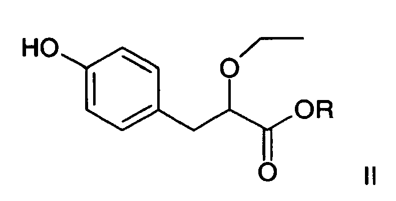 in which R is as previously defined with a compound of formula III 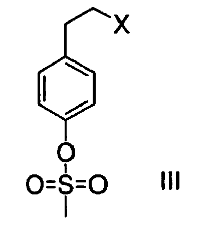 wherein X is a suitable leaving group in the presence of a base and using water as a diluent.

2. A process for the preparation of a compound of formula I 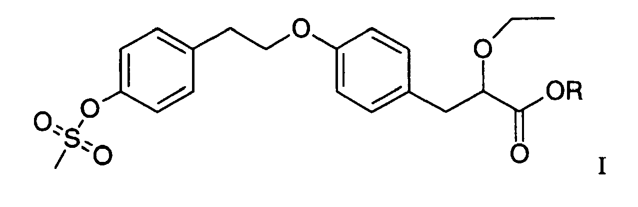 in which R represents H which comprises reacting a compound of formula II 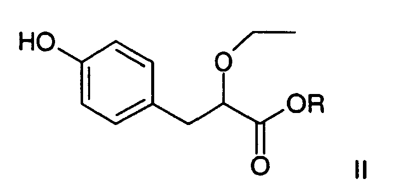 in which R represents an acid protecting group with a compound of formula III 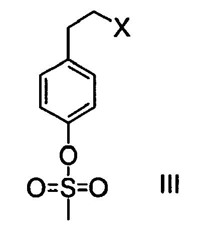 wherein X is a suitable leaving group in the presence of a base and using water as the diluent to give a compound of formula I in which R is an acid protecting group and then removing the protecting group to give a compound of formula I in which R is H.

3. A process according to claim 2 in which the acid protecting group is removed by hydrolysis.

4. A process according to any preceding claim in which R is H, benzyl or a (1-4C)alkyl group.

5. A process according to any preceding claim in which X is halo, an optionally substituted phenylsulfonyloxy group or an alkylsulphonyloxy group.

6. A process according to any preceding claim in which the base is selected from carbonates, hydrogen carbonates or hydroxides of alkali metals.

7. A process according to any preceding claim in which the compound of formula I is the S enantiomer.

## Patentansprüche

1. Verfahren zur Herstellung einer Verbindung der Formel I 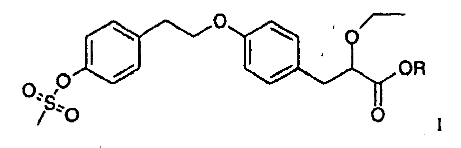 worin R für H oder eine Säureschutzgruppe steht, bei dem man eine Verbindung der Formel II 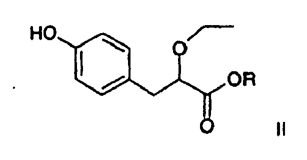 worin R die oben angegebene Bedeutung besitzt, in Gegenwart einer Base und unter Verwendung von Wasser als Verdünnungsmittel mit einer Verbindung der Formel III 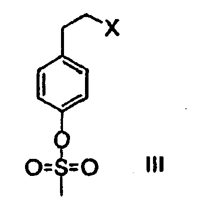 worin X für eine geeignete Abgangsgruppe steht, umsetzt.

2. Verfahren zur Herstellung einer Verbindung der Formel I 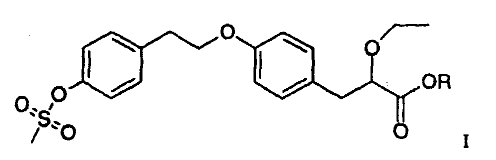 worin R für H steht, bei dem man eine Verbindung der Formel II 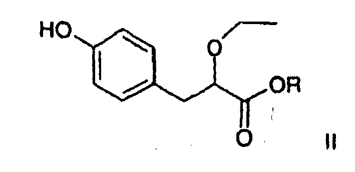 worin R für eine Säureschutzgruppe steht, in Gegenwart einer Base und unter Verwendung von Wasser als Verdünnungsmittel mit einer Verbindung der Formel III 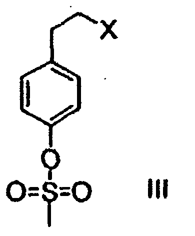 worin X für eine geeignete Abgangsgruppe steht, zu einer Verbindung der Formel I, worin R für eine Säureschutzgruppe steht, umsetzt und dann die Schutzgruppe abspaltet, wobei man eine Verbindung der Formel I, worin R für H steht, erhält.

3. Verfahren nach Anspruch 2, bei dem man die Säureschutzgruppe durch Hydrolyse abspaltet.

4. Verfahren nach einem der vorhergehenden Ansprüche, bei dem R für H, Benzyl oder eine C₁₋₄-Alkylgruppe steht.

5. Verfahren nach einem der vorhergehenden Ansprüche, bei dem X für Halogen, eine gegebenenfalls substituierte Phenylsulfonyloxygruppe oder eine Alkylsulfonyloxygruppe steht.

6. Verfahren nach einem der vorhergehenden Ansprüche, bei dem man die Base unter Carbonaten, Hydrogencarbonaten oder Hydroxiden von Alkalimetallen auswählt.

7. Verfahren nach einem der vorhergehenden Ansprüche, bei dem es sich bei der Verbindung der Formel I um das S-Enantiomer handelt.

## Revendications

1. Procédé de préparation d'un composé de formule I 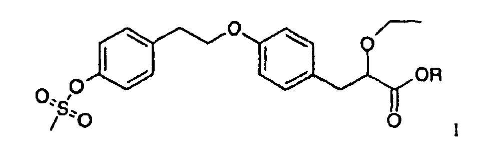 dans laquelle R représente un H ou un groupe protecteur d'acide, comprenant la réaction d'un composé de formule II 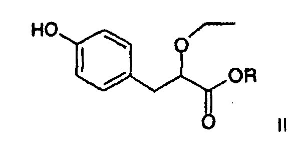 dans laquelle R est tel que défini précédemment, avec un composé de formule III 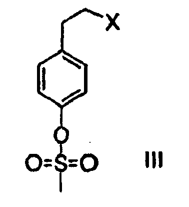 dans laquelle X est un groupe partant approprié, en présence d'une base et en utilisant de l'eau en tant que diluant.

2. Procédé de préparation d'un composé de formule I 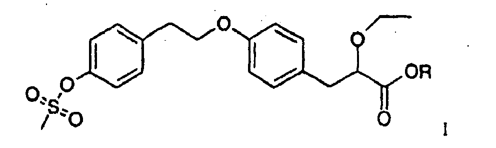 dans laquelle R représente un H, comprenant la réaction d'un composé de formule II 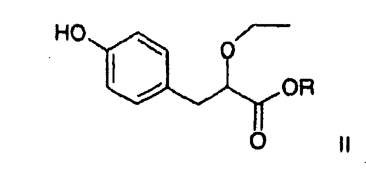 dans laquelle R représente un groupe protecteur d'acide, avec un composé de formule III 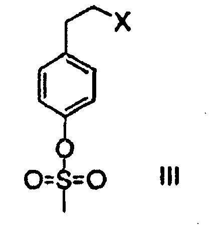 dans laquelle X est un groupe partant approprié, en présence d'une base et en utilisant de l'eau en tant que diluant, pour donner lieu à un composé de formule I dans laquelle R est un groupe protecteur d'acide, puis l'élimination du groupe protecteur pour donner lieu à un composé de formule I dans laquelle R est un H.

3. Procédé selon la revendication 2, dans lequel le groupe protecteur d'acide est éliminé par hydrolyse.

4. Procédé selon l'une quelconque des revendications précédentes, dans lequel R est un H, un benzyle ou un groupe alkyle en C₁-C₄.

5. Procédé selon l'une quelconque des revendications précédentes, dans lequel X est un halogéno, un groupe phénylsulfonyloxy éventuellement substitué ou un groupe alkylsulfonyloxy.

6. Procédé selon l'une quelconque des revendications précédentes, dans lequel la base est choisie parmi des carbonates, des hydrogénocarbonates ou des hydroxydes de métaux alcalins.

7. Procédé selon l'une quelconque des revendications précédentes, dans lequel le composé de formule I est l'énantiomère S.
